# EUROPÄISCHE PATENTANMELDUNG

(11) **EP 1 188 737 A1**
(43) Veröffentlichungstag der Anmeldung: **20.03.2002**
(21) Anmeldenummer: 01120370.0
(22) Anmeldetag: 25.08.2001
(51) Int. Cl.: C07C 45/85, C07C 37/86, C07C 45/53, C07C 37/08, C07C 49/08

(54) **Verfahren zur Aufarbeitung von Mesityloxid aufweisenden Stoffströmen**

(30) Priorität: 14.09.2000 DE 10045355
(71) Anmelder: Phenolchemie GmbH & Co. KG, 45966 Gladbeck (DE)
(72) Erfinder: Gerlich, Otto, Dr., 45966 Gladbeck (DE); Van Barneveld, Heinrich, Dr., 46244 Bottrop (DE); Jordan, Wilfried, Dr., 46282 Dorsten (DE)
(74) Vertreter: Sternagel, Fleischer, Godemeyer & Partner Patentanwälte

(57) **Zusammenfassung**

Es wird ein Verfahren zur Aufarbeitung von Mesityloxid aufweisenden Stoffströmen beansprucht. Insbesondere wird ein Verfahren zur Gewinnung von Aceton aus Mesityloxid, welches im Prozess der Herstellung von Aceton und Phenol aus Cumol nach dem Hock Verfahren anfällt, beansprucht. Auf Grund der Prozessführung beim Hock Verfahren wandelt sich Aceton teilweise zu Diacetonalkohol und weiter zu Mesityloxid um. Das Mesityloxid liegt in den verschiedenen beim Phenolprozess anfallenden Fraktionen oder Phasen allerdings nur in relativ geringen Konzentrationen vor, so dass eine Aufarbeitung und Weiterverarbeitung des Mesityloxid wirtschaftlich nicht sinnvoll ist. Üblicherweise werden die Mesityloxid und weitere weniger wertvolle Nebenprodukte enthaltenden Stoffströme unter Nutzung der entstehenden Wärmeenergie verbrannt.

Mit Hilfe der vorliegenden Erfindung ist es möglich zumindest einen Teil des Mesityloxids durch Anlagerung von Wasser wieder in Aceton zu überführen. Das so hergestellte Rohaceton kann relativ einfach auf dem Fachmann bekannte Weise von den Mesityloxid und Nebenprodukte aufweisenden Stoffströmen abgetrennt werden und im Gesamtprozess der Phenolherstellung der dort stattfindenden Acetonaufarbeitung zugeführt werden.

## Beschreibung

Die vorliegende Erfindung betrifft ein Verfahren zur Aufarbeitung von Mesityloxid aufweisenden Stoffströmen.

Im besonderen betrifft die vorliegende Erfindung ein Verfahren zur Gewinnung von Aceton aus Mesityloxid enthaltenden Stoffströmen, die im Prozess der säurekatalysierten Spaltung von Cumolhydroperoxid anfallen, durch saure oder alkalische Spaltung von Mesityloxid.

Im Prozess zur Gewinnung von Phenol und Aceton ausgehend von Cumol nach dem klassischen Hock-Verfahren, der von großer wirtschaftlicher Bedeutung ist, fallen bei der säurekatalysierten Spaltung von Cumolhydroperoxid große Mengen Aceton an. Häufig wird das Aceton durch Behandlung mit alkalischen Reagenzien von unerwünschten Nebenprodukten befreit. Unter diesen sauren bzw. alkalischen Reaktionsbedingungen kommt es zu einer Reaktion des Acetons mit sich selbst, und es bildet sich in einer ersten Stufe Diacetonalkohol, der unter Wasserabgabe Mesityloxid bilden kann oder auch wieder in Aceton zerfallen kann.

Bei der Phenolherstellung durch säurekatalysierte Spaltung von Cumolhydroperoxid entstehen im Prozess neben Phenol und Aceton weitere Nebenprodukte und/oder Verunreinigungen, wie z.B. α-Methylstyrol, Cumol und weitere Verbindungen, die in der Spaltproduktphase vorhanden sind. Diese müssen von dem Phenol und dem Aceton abgetrennt werden. Üblicherweise erfolgt dies durch Extraktion und/oder Destillation. Bei der destillativen Aufarbeitung der Spaltproduktphase fallen neben Fraktionen, die hauptsächlich Phenol oder überwiegend Aceton enthalten, auch Fraktionen an, die unter anderem Mesityloxid aufweisen. Diese Fraktionen werden üblicherweise wiederum destillativ aufgearbeitet und man erhält Fraktionen, die zum Teil mehr als 50 Gew.-% Mesityloxid aufweisen. Eine Nutzung dieser Fraktionen war bislang wirtschaftlich nicht sinnvoll, weshalb diese Fraktionen üblicherweise unter Nutzung der Wärmeenergie im gesamten Phenolprozess verbrannt wurden.

Durch das anfallende Mesityloxid kommt es zu Ausbeuteverlusten an Aceton, die die Wirtschaftlichkeit der Herstellung von Phenol und Aceton nach dem Hock-Verfahren beeinträchtigen.

Eine Nutzung des Mesityloxids zur Herstellung von Wertstoffen, wie z.B. Methylisobutylketon, ist mit Stoffströmen, die neben Mesityloxid weitere Verbindungen aufweisen, nicht sinnvoll möglich.

Es stellte sich damit die Aufgabe, ein Verfahren zur Aufarbeitung von Mesityloxid enthaltenden Stoffströmen bereitzustellen.

Überraschenderweise wurde gefunden, dass durch Nutzung der Rückreaktion zur Bildungsreaktion von Mesityloxid aus Aceton, das Aceton als Wertstoff wirtschaftlich sinnvoll aus Mesityloxid, welches in Mesityloxid aufweisenden Stoffströmen vorliegt, zurückgewonnen werden kann und somit die Ausbeuteverluste an Aceton, welche durch die Bildung von Mesityloxid aus Aceton auftreten, im Gesamtprozess der Phenolherstellung verringert werden kann.

Gegenstand der vorliegenden Erfindung ist deshalb ein Verfahren zur Aufarbeitung von Mesityloxid aufweisenden Phasen, welches dadurch gekennzeichnet ist, dass zumindest ein Teil des Mesityloxids durch Anlagerung von Wasser in einem sauren oder basischen Medium oder durch Reaktion mit Wasser an einem sauren Katalysator in Aceton gespalten wird.

Ebenfalls Gegenstand der vorliegenden Erfindung ist deshalb ein Verfahren zur Herstellung von Phenol und Aceton aus Cumol nach dem Hock'schen Verfahren, bei welchem durch Oxidation von Cumol Cumolhydroperoxid hergestellt wird und durch anschließende säurekatalysierte Spaltung von Cumolhydroperoxid eine Spaltproduktphase erhalten wird, welches dadurch gekennzeichnet ist, dass in Mesityloxid aufweisenden Phasen, die bei der destillativen Aufbereitung der Spaltproduktphase anfallen, durch Anlagerung von Wasser an Mesityloxid in einem sauren oder basischen Medium oder durch Anlagerung von Wasser an Mesityloxid an einem sauren Katalysator zumindest ein Teil des Mesityloxids in Aceton umgewandelt wird.

Mit Hilfe des erfindungsgemäßen Verfahrens lassen sich Mesityloxid aufweisende Stoffströme, die nicht als Ausgangsprodukt für die Herstellung von Folgeprodukten, wie z.B. Methylisobutylketon, geeignet sind, aufarbeiten. Erfindungsgemäß kann zumindest ein Teil des Mesityloxids durch Reaktion mit Wasser in Aceton umgewandelt werden, welches relativ einfach, z.B. durch Destillation, vom Reaktionsgemisch abgetrennt werden kann und somit einer Weiterverarbeitung bzw. -verwendung zur Verfügung gestellt werden kann.

Ein weitere Vorteil des erfindungsgemäßen Verfahrens besteht darin, dass die Wirtschaftlichkeit des Prozesses der Herstellung von Phenol nach dem Hock-Verfahren wesentlich erhöht werden kann. Durch Anwendung des erfindungsgemäßen Verfahrens lässt sich die Menge des aus dem Prozess ausgeschleusten Mesityloxids verringern, so dass die Ausbeuteverluste an Aceton geringer ausfallen als bei herkömmlichen Prozessen.

Das erfindungsgemäße Verfahren bedient sich der Umkehr der bekannten Reaktion, bei welcher zwei Moleküle Aceton unter sauren oder basischen Bedingungen in einer Aldolreaktion zu Diacetonalkohol abreagieren. Dieser reagiert unter Wasserabspaltung weiter zu Mesityloxid. Zur Verringerung des Anteils an Mesityloxid in Mesityloxid aufweisenden Stoffströmen wird die Reaktion erfindungsgemäß umgekehrt. Zu diesem Zweck wird zu einem Mesityloxid aufweisenden Stoffstrom Wasser oder ein Wasser aufweisendes saures oder basisches Medium zugemischt, und dieses Reaktionsgemisch wird in basischer oder saurer Umgebung thermisch behandelt. Wird dem Mesityloxid aufweisenden Stoffstrom nur Wasser zugemischt, so muss die thermische Behandlung in Anwesenheit eines sauren Katalysators durchgeführt werden. Ein solcher Katalysator kann z.B. ein Zeolith oder ein saures Ionenaustauscherharz sein.

Vorzugsweise wird dem Mesityloxid aufweisenden Stoffstrom als saures Medium eine Mineralsäure oder eine organische Säure, wie z.B. Salz-, Schwefel-, oder Essigsäure, zugemischt. Ganz besonders bevorzugt wird dem zu behandelnden Stoffstrom als saures Medium verdünnte Schwefelsäure zugemischt. Als basische Medien können Alkalilaugen, wie z.B. Natronlauge, Erdalkalilaugen oder auch organische Phasen, welche Verbindungen mit basischen Eigenschaften aufweisen, wie z.B. Amine oder Phenolate, eingesetzt werden. Ganz besonders bevorzugt wird dem zu behandelnden Stoffstrom eine 1 bis 10 %-ige, vorzugsweise eine ca. 4 %-ige, Natronlauge zugemischt. Vorzugsweise wird dem Mesityloxid aufweisenden Stoffstrom so viel eines sauren oder basischen Mediums zugemischt, dass das Reaktionsgemisch aus Mesityloxid aufweisendem Stoffstrom und saurem oder basischem Medium von 5 bis 15 Volumen-%, bezogen auf das Gesamtvolumen des Reaktionsgemisches, des sauren oder basischen Mediums aufweist.

Das Reaktionsgemisch, welches sehr gut durchmischt sein sollte, wird zur thermischen Behandlung auf eine Temperatur von 60 bis 95 °C, vorzugsweise auf eine Temperatur von 80 bis 90 °C erhitzt. Beim Erhitzen reagiert das in dem Reaktionsgemisch vorhandene Mesityloxid unter den sauren oder basischen Bedingungen des eingesetzten Mediums oder am sauren Katalysator mit dem Wasser zu Diacetonalkohol, welches in zwei Acetonmoleküle zerfällt. Das Aceton kann auf bekannte Weise, z.B. durch Destillation, aus dem Reaktionsgemisch entfernt werden.

Das erfindungsgemäße Verfahren kann kontinuierlich oder diskontinuierlich durchgeführt werden. Vorzugsweise wird das erfindungsgemäße Verfahren diskontinuierlich durchgeführt. Bei der Durchführung des Verfahrens kann es vorteilhaft sein, Wasser, welches bei der Reaktion verbraucht wurde, nachzudosieren. Ebenfalls kann es vorteilhaft sein, so viel des Mesityloxid aufweisenden Stoffstroms zum Reaktionsgemisch zuzudosieren, dass das in Diacetonalkohol bzw. Aceton umgewandelte Mesityloxid ersetzt wird.

Das erfindungsgemäße Verfahren kann auf alle Mesityloxid aufweisenden Stoffströme angewendet werden. Vorzugsweise werden Mesityloxid aufweisende Stoffströme eingesetzt, die mehr als 20 Gew.-% Mesityloxid, bevorzugt von 30 bis 90 Gew.-% Mesityloxid und ganz besonders bevorzugt von 50 bis 85 Gew.-% Mesityloxid aufweisen. Es kann vorteilhaft sein, das Mesityloxid in Mesityloxid aufweisenden Stoffströmen z.B. durch Destillation oder andere geeignete Maßnahmen anzureichern.

Das erfindungsgemäße Verfahren wird im folgenden beispielhaft anhand der Aufarbeitung einer Spaltproduktphase, welche bei der Hock'schen Synthese, insbesondere bei der säurekatalysierten Spaltung von Cumolhydroperoxid, entsteht, beschrieben, ohne darauf beschränkt zu sein.

Bei der in bekannter Weise durchgeführten Hock'schen Synthese wird aus Cumol und Sauerstoff Cumolhydroperoxid hergestellt. Dieses wird in einer Spaltreaktion säurekatalysiert in Phenol und Aceton aufgespalten. Bei dieser Spaltreaktion finden jedoch auch Nebenreaktionen, die zu Nebenprodukten führen, statt, so dass in der Spaltproduktphase neben Phenol und Aceton weitere Verbindungen sowie der verwendete Katalysator vorhanden sind.

Üblicherweise erfolgt die Aufarbeitung des organischen Teils dieser Spaltproduktphasen dadurch, dass die verschiedenen Inhaltsstoffe dieser Phase destillativ und/oder durch Extraktion getrennt werden. Die destillative Trennung erfolgt üblicherweise in mehreren hintereinander geschalteten Destillationskolonnen. Auf Grund ähnlicher Siedetemperaturen oder durch chemische Attraktionskräfte enthalten die bei diesen Destillationen erhaltenen Fraktionen oder Ströme häufig nicht nur eine chemische Verbindung sondern mehrere. Diese Fraktionen oder Ströme werden häufig weiteren Aufarbeitungsschritten zugeführt, welche im wesentlichen wieder aus Destillationsschritten bestehen.

Erfindungsgemäß werden Stoffströme, wie z.B. Fraktionen oder Ströme, die Mesityloxid aufweisen und die aus der direkten Aufarbeitung der Spaltproduktphase, insbesondere aus der Aufarbeitung des Acetons, aber auch aus Seitenströmen oder Fraktionen, die bei der Aufarbeitung bzw. Rückgewinnung der Nebenprodukte anfallen, stammen können, vereinigt und destillativ wiederum so aufbereitet, dass eine Fraktion oder ein Strom erhalten wird, der/die zumindest 20 Gew.-%, vorzugsweise von 30 bis 90 Gew.-%, besonders bevorzugt von 50 bis 85 Gew.-% Mesityloxid enthält. Diese Aufarbeitung kann kontinuierlich oder diskontinuierlich, vorzugsweise kontinuierlich erfolgen.

Die so erhaltenen Fraktionen oder Ströme, aber auch andere Phasen, welche zumindest 20 Gew.-%, vorzugsweise von 30 bis 90 Gew.-%, besonders bevorzugt von 50 bis 85 Gew.-% Mesityloxid aufweisen, werden erfindungsgemäß mit Wasser in einem sauren oder basischen Medium oder an einem sauren Katalysator durch thermische Behandlung zur Reaktion gebracht. Dabei lagert sich Wasser an das Mesityloxid an und es entsteht Diacetonalkohol. Dieser Diacetonalkohol spaltet sich in zwei Moleküle Aceton auf, so dass pro Mol umgesetzten Mesityloxids zwei Mol Aceton gebildet werden. Vorzugsweise findet die thermische Behandlung und die Reaktion, bei einer Temperatur von 80 bis 90 °C und einem Druck von 800 bis 1100 mbar statt.

Die sauren oder basischen Medien können z.B. Säuren oder Basen, oder Säuren oder Basen enthaltende Lösungen sein. Vorzugsweise werden als Säuren anorganische Mineralsäuren, wie z.B. Salz- oder Schwefelsäure oder wasserlösliche organische Säuren, wie z.B. Essigsäure, eingesetzt. Ganz besonders bevorzugt wird als saures Medium verdünnte Schwefelsäure eingesetzt. Als Basen können organische oder anorganische Basen, wie z.B. organische Amine, Ammoniak oder Alkali- oder Erdalkalilaugen eingesetzt werden. Ganz besonders bevorzugt wird als basisches Medium Natronlauge, vorzugsweise eine 1 bis 10 %-ige Natronlauge und besonders bevorzugt eine 4 %-ige Natronlauge eingesetzt. Vorzugsweise weisen die sauren Medien einen pH-Wert von 1 bis 3 auf. Die basischen Medien weisen vorzugsweise einen pH-Wert von 8 bis 10 auf. Das für die Reaktion benötigte Wasser kann aus den sauren oder basischen Medien stammen oder extra zum Reaktionsgemisch hinzugegeben werden. Als saure Katalysatoren können z.B. Zeolithe oder saure Ionentauscher eingesetzt werden. Werden solche Katalysatoren für die Reaktion verwendet, muss pro Mol Mesityloxid, welches gespalten werden soll, zumindest ein Mol Wasser dem Reaktionsgemisch zugegeben werden. Vorzugsweise wird die Reaktion bei der Verwendung von Katalysatoren mit einem Überschuss an Wasser durchgeführt.

Erfindungsgemäß werden die sauren oder basischen Medien in innigen Kontakt mit dem zu behandelnden Mesityloxid aufweisenden Stoffstrom gebracht. Da dieser Stoffstrom neben Mesityloxid häufig weitere organische Verbindungen enthält, welche ebenfalls schlecht oder gar nicht wasserlöslich sind, muss zum Herstellen des innigen Kontaktes dieses Stoffstroms mit dem sauren oder basischen Medium für eine gute Durchmischung gesorgt werden. Diese kann auf allgemein bekannte Weise erfolgen. Vorzugsweise erfolgt das Durchmischen mit Hilfe von Pumpen, Rührern oder beim Thermosyphonumlauf in einem Verdampfer.

Das aus durchmischtem, zu behandelnden, Mesityloxid aufweisenden Stoffstrom und saurem oder basischem Medium bestehende Reaktionsgemisch wird vorzugsweise während des Durchmischens oder nach dem Durchmischen, vorzugsweise während des Durchmischens, erhitzt bzw. thermisch behandelt. Das Durchmischen und Erhitzen kann in einer oder mehreren Apparaturen erfolgen. Vorzugsweise erfolgt das Durchmischen und Erhitzen des Gemisches in einer Apparatur. Die Apparaturen können z.B. Reaktoren, Reaktionskolonnen, Destillationskolonnen, Mischbehälter oder ähnliches sein. Vorzugsweise wird als Apparatur eine Destillations- oder Reaktionskolonne, besonders bevorzugt eine Destillationsblase, verwendet. In dieser Apparatur findet die Reaktion, bei welcher Wasser an Mesityloxid angelagert wird und das Mesityloxid in zwei Aceton gespalten wird, bei einer Temperatur von 80 bis 90 °C und bei einem Druck von 800 bis 1100 mbar statt.

Da bei der Reaktion Wasser verbraucht wird, ist es vorteilhaft, wenn Wasser in die Apparatur, in welcher die Reaktion stattfindet, wie z.B. in dem Reaktionsbehälter oder der Reaktions- bzw. Destillationskolonne oder -blase, nachdosiert wird. Es wird vorzugsweise so viel Wasser nachdosiert, dass die Laugenkonzentration im Gemisch etwa 4 % beträgt. Vorzugsweise wird außerdem so viel Mesityloxid aufweisender Stoffstrom oder Fraktion zum Gemisch nachdosiert, dass das zu Aceton umgesetzte Mesityloxid ersetzt wird.

Das bei der Reaktion aus Mesityloxid entstandene Aceton kann von den übrigen Komponenten des Gemisches auf verschiedenste dem Fachmann bekannten Weisen abgetrennt werden. Vorzugsweise wird das Aceton destillativ von dem Gemisch getrennt. Das Reaktionsgemisch kann entweder in eine Destillationskolonne eingebracht und destillativ getrennt werden oder die Reaktion selbst kann in einer Destillations- bzw. Reaktionskolonne oder -blase durchgeführt werden, so dass während der Reaktion entstehendes Aceton vom restlichen Reaktionsgemisch abdestilliert werden kann.

In allen Fällen wird das Aceton, vorzugsweise am Kopf der Reaktions- oder Destillationskolonne oder -blase, gasförmig abgenommen. Es kann vorteilhaft sein, einen Teil des abgenommenen Acetons zu kondensieren und als Rücklauf in die Kolonne zurückzuspeisen. Auf diese Weise lässt sich vermeiden, dass größere Mengen an unerwünschten Komponenten mit dem Aceton aus der Kolonne ausgeschleust werden. Das so gewonnene Rohaceton kann einer weiteren Aufarbeitung zugeführt werden. Vorzugsweise wird das erfindungsgemäß erhaltene Rohaceton mit den Acetonströmen, die bei der Aufarbeitung der Spaltproduktphase anfallen, vereinigt. Diese vereinigten Acetonphasen werden dann gemeinsam aufgearbeitet.

Das im Sumpf der Destillationsblase bzw. -kolonne vorliegende Gemisch, welches nicht umgesetztes Mesityloxid, Cumol und weitere Produkte und/oder Nebenprodukte aus dem Prozess der Phenolherstellung enthalten kann wird einer Aufarbeitung oder Verbrennung zugeführt. Vorzugsweise erfolgt die Aufarbeitung dadurch, dass das aus dem Sumpf der Destillationskolonne bzw. -blase ausgeschleuste Gemisch auf bekannte Weise in eine organische und eine wässrige Phase getrennt werden. Die wässrige Phase, welche in der Hauptsache das bei der Reaktion eingesetzte basische oder saure Medium aufweist, kann in den Prozess der Aufarbeitung von Mesityloxid aufweisenden Stoffströmen zurückgeführt werden. Die organische Phase, welche Mesityloxid, Cumol und organische Produkte, Nebenprodukte oder Edukte aus dem Prozess der Phenolherstellung aufweisen kann, wird je nach Zusammensetzung entweder einer thermischen Verwertung zugeführt oder in den Prozess der Aufarbeitung der Spaltproduktphase zurückgeführt, um evtl. noch vorhandenes Cumol zurückgewinnen zu können.

Die Umsetzung der Mesityloxid aufweisenden Phase mit Wasser in einem sauren oder organischen Medium und die anschließende destillative Aufarbeitung kann diskontinuierlich oder kontinuierlich, vorzugsweise diskontinuierlich erfolgen.

Die Reaktion der Umsetzung von Mesityloxid mit Wasser und die anschließende destillative Aufarbeitung können in unterschiedlichen Apparaturen nacheinander durchgeführt werden, z.B. in einem Reaktionsbehälter und in einer separaten Destillationsapparatur oder in einer einzigen Apparatur durchgeführt werden. Vorzugsweise wird die Reaktion der Umsetzung des Mesityloxids mit Wasser und die anschließende destillative Aufarbeitung in einer einzigen Apparatur, vorzugsweise in einer Destillationskolonne oder -blase durchgeführt.

In einer besonderen Ausführungsart des erfindungsgemäßen Verfahrens werden nicht saure oder basische Medien sondern saure Katalysatoren bei der Durchführung der Reaktion verwendet. Als saure Katalysatoren eignen sich z.B. saure Ionenaustauscherharze oder Zeolithe bzw. Schichtsilikate, insbesondere Schichtsilikate bzw. Zeolithe in der H-Form. Bei dieser Ausführungsart des erfindungsgemäßen Verfahren wird Wasser und der zu behandelnde, Mesityloxid aufweisende Stoffstrom gemischt, erhitzt und mit dem Katalysator in Kontakt gebracht. Wiederum muss für eine ausreichende Durchmischung des Wassers mit dem zu behandelnden, Mesityloxid aufweisenden Stoffstrom gesorgt werden. Dies kann ebenfalls auf allgemein bekannte Weise, z.B. wie oben beschrieben, erfolgen. Die Reaktion und die anschließende destillative Aufarbeitung können wiederum in einer einzigen Apparatur, wie z.B. einer Destillationsblase, durchgeführt werden.

Je nach Temperaturstabilität des eingesetzten Katalysators kann es auch vorteilhaft sein, die Reaktion und die anschließende destillative Aufarbeitung in zwei unterschiedlichen Behältern durchzuführen. Das gut durchmischte Reaktionsgemisch aus Wasser und zu behandelnder, Mesityloxid aufweisender Phase wird in diesem Fall auf eine Temperatur von 60 bis 80 °C gebracht und in eine Apparatur, z.B. einen Ionentauscher, der mit einem sauren Ionenaustauscherharz als Katalysator, wie z.B. Levatit SPC 108, gefüllt ist, gefahren. Das in der Apparatur bzw. dem Ionenaustauscher behandelte Reaktionsgemisch wird anschließend in eine Destillationskolonne gefahren, in welcher, wie oben beschrieben, das entstandene Aceton vom Rest des Reaktionsgemisches destillativ abgetrennt wird. Es kann vorteilhaft sein, einen Teil des im Ionenaustauscher behandelten Gemisches und/oder einen Teil des im Sumpf der Destillationskolonne angefallenen Restes des Reaktionsgemisches wieder in den Ionenaustauscher zurückzuführen. Als Katalysator aufweisende Apparatur kann nicht nur ein Ionenaustauscher verwendet werden, sondern es können auch z.B. Fest- oder Fließbettreaktoren oder ähnliche Apparaturen eingesetzt werden.

Auch diese Ausführungsart des erfindungsgemäßen Verfahrens kann kontinuierlich oder diskontinuierlich durchgeführt werden. Vorzugsweise wird diese Ausführungsart des erfindungsgemäßen Verfahrens diskontinuierlich durchgeführt.

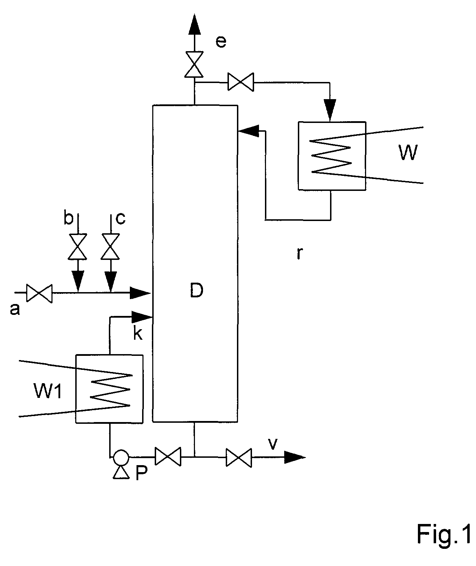

In Figur 1 ist beispielhaft eine Ausführungsart des erfindungsgemäßen Verfahrens dargestellt, ohne dass das Verfahren auf diese beschränkt ist.

Die zu behandelnde, Mesityloxid enthaltende Phase wird über Leitung a in die Destillationskolonne **D** gefahren. Über Leitung **b** kann Lauge oder Säure der zu behandelnden Phase beigemischt werden. Über Leitung **c** kann Wasser zu der zu behandelnden Phase zugemischt werden. Das Gemisch aus zu behandelnder Phase, Lauge oder Säure und Wasser wird in Kolonne **D** erhitzt. Das bei der Reaktion des Mesityloxids mit Wasser entstehende Aceton wird über Kopf dampfförmig abdestilliert, wobei es entweder direkt über Leitung **e** einer weiteren Aufarbeitung zugeführt wird oder zumindest teilweise über den Wärmetauscher **W**, in welchem das Aceton kondensiert wird, und Leitung **r** in die Destillationskolonne zurückgeführt wird. Die nicht siedenden Inhaltsstoffe, wie z.B. nicht umgesetztes Mesityloxid, aber auch weitere in der zu behandelnden Phase enthaltenen Nebenprodukte oder Verunreinigungen aus der Phenolherstellung bzw. Acetonaufarbeitung, wie z.B. Cumol sowie die Lauge, die Säure und oder das Wasser reichern sich im Sumpf der Kolonne an. Aus dem Sumpf der Kolonne wird dieses Gemisch mit Hilfe der Pumpe **P** über Leitung **k** und den Wärmetauscher **W1** in die Destillationskolonne wieder eingespeist. Ein Teil des am Sumpf abgenommenen Gemisches kann über Leitung **v** nach Phasentrennung einer weiteren Aufarbeitung oder der thermischen Verwertung zugeführt werden.

Das erfindungsgemäße Verfahren wird durch das folgende Beispiel näher erläutert, ohne darauf beschränkt zu sein.

### Beispiel 1:

In eine Destillationsblase wurden 78,8 t einer Ketonefraktion, die 51 % Mesityloxid aufwies, und so viel einer 4 %-igen Natronlauge eingebracht, dass ein Gemisch entstand, welches ca. 10 Vol.-% Natronlauge aufwies. Durch Mischen in einer Pumpe wurden die Komponenten in innigen Kontakt miteinander gebracht und das Gemisch wurde auf eine Temperatur von 85 °C erhitzt. Das erhaltene Aceton wurde über den Kopf der Destillationsblase abdestilliert, wobei ein Teil des abdestillierten Acetons als Rücklauf in die Destillationsblase wieder eingespeist wurde.

Da während der Reaktion Wasser verbraucht wurde, wurde laufend 0,5 t/h Wasser nachdosiert, so dass die Laugenkonzentration bei einem Wert von etwa 4 % gehalten wurde. Gleichzeitig wurde 2,5 t/h Ketonefraktion nachdosiert, so dass das umgesetzte Mesityloxid ersetzt wurde.

Die Auswertung dieses Versuches zeigte, dass von den in den 78,8 t Ketonefraktion vorhandenen 51 % Mesityloxid ca. 75 % zu Aceton umgesetzt wurden, was einer Acetonmenge von 39,5 t entspricht. Dieser Versuch zeigte, dass mit dem erfindungsgemäßen Verfahren die Menge an Ketonfraktion, welche nur zur thermischen Nutzung verwendet werden kann, wesentlich verringert werden kann.

## Patentansprüche

1. Verfahren zur Aufarbeitung von Mesityloxid aufweisenden Stoffströmen,
**dadurch gekennzeichnet,**
**dass** zumindest ein Teil des Mesityloxids durch Anlagerung von Wasser in einem sauren oder basischen Medium oder durch Reaktion mit Wasser an einem sauren Katalysator in Aceton gespalten wird.

2. Verfahren zur Herstellung von Phenol und Aceton aus Cumol nach dem Hock'schen Verfahren, bei welchem durch Oxidation von Cumol Cumolhydroperoxid hergestellt wird und durch anschließende säurekatalysierte Spaltung von Cumolhydroperoxid eine Spaltproduktphase erhalten wird,
**dadurch gekennzeichnet,**
**dass** in Mesityloxid aufweisenden Stoffströmen, die bei der destillativen Aufbereitung der Spaltproduktphase anfallen, durch Anlagerung von Wasser an Mesityloxid in einem sauren oder basischen Medium oder durch Anlagerung von Wasser an Mesityloxid an einem sauren Katalysator zumindest ein Teil des Mesityloxids in Aceton umgewandelt wird.

3. Verfahren nach einem der Ansprüche 1 oder 2,
**dadurch gekennzeichnet,**
**dass** die Mesityloxid aufweisenden Stoffströme zur Anreicherung des Mesityloxids destillativ behandelt werden.

4. Verfahren nach zumindest einem der Ansprüche 1 bis 3,
**dadurch gekennzeichnet,**
**dass** die Mesityloxid aufweisenden Stoffströme zumindest 20 Gew.-% Mesityloxid aufweisen.

5. Verfahren nach Anspruch 4,
**dadurch gekennzeichnet,**
**dass** die Mesityloxid aufweisenden Stoffströme von 50 bis 85 Gew.-% Mesityloxid aufweisen.

6. Verfahren nach zumindest einem der Ansprüche 1 bis 5,
**dadurch gekennzeichnet,**
**dass** dem Mesityloxid aufweisenden Stoffstrom ein basisches oder saures Medium hinzugefügt wird.

7. Verfahren nach Anspruch 6,
**dadurch gekennzeichnet,**
**dass** als saures Medium eine Mineralsäure oder eine organische Säure verwendet wird.

8. Verfahren nach Anspruch 6,
**dadurch gekennzeichnet,**
**dass** als basisches Medium eine Alkalilauge oder eine organische Phase, die Phenolate oder Amine aufweist, verwendet wird.

9. Verfahren nach Anspruch 6,
**dadurch gekennzeichnet,**
**dass** als basisches Medium eine 1 bis 10 %-ige Natronlauge verwendet wird.

10. Verfahren nach zumindest einem der Ansprüche 1 bis 9,
**dadurch gekennzeichnet,**
**dass** das Reaktionsgemisch aus Mesityloxid aufweisendem Stoffstrom und saurem oder basischem Medium von 5 bis 15 Volumen-%, bezogen auf das Gesamtvolumen des Reaktionsgemisches, des sauren oder basischen Mediums aufweist.

11. Verfahren nach Anspruch 10,
**dadurch gekennzeichnet,**
**dass** das Reaktionsgemisch aus Mesityloxid aufweisendem Stoffstrom und saurem oder basischem Medium 10 Volumen-%, bezogen auf das Gesamtvolumen des Reaktionsgemisches, des sauren oder basischen Mediums aufweist.

12. Verfahren nach zumindest einem der Ansprüche 1 bis 5,
**dadurch gekennzeichnet,**
**dass** der Mesityloxid aufweisende Stoffstrom mit Wasser vermischt wird und dieses Reaktionsgemisch in Anwesenheit eines sauren Katalysators thermisch behandelt wird.

13. Verfahren nach Anspruch 12,
**dadurch gekennzeichnet,**
**dass** als Katalysator ein Zeolith oder ein saures Ionenaustauscherharz verwendet wird.

14. Verfahren nach zumindest einem der Ansprüche 1 bis 13,
**dadurch gekennzeichnet,**
**dass** das Reaktionsgemisch bei einer Temperatur von 60 bis 95 °C thermisch behandelt wird.

15. Verfahren nach Anspruch 14,
**dadurch gekennzeichnet,**
**dass** das Reaktionsgemisch bei einer Temperatur von 80 bis 90 °C thermisch behandelt wird.

16. Verfahren nach zumindest einem der Ansprüche 1 bis 15,
**dadurch gekennzeichnet,**
**dass** das bei der thermischen Behandlung des Reaktionsgemisches entstehende Aceton destillativ aus dem Reaktionsgemisch entfernt wird.

17. Verfahren nach zumindest einem der Ansprüche 1 bis 16,
**dadurch gekennzeichnet,**
**dass** die Reaktion, bei welcher Wasser an das Mesityloxid angelagert wird und dieses in Aceton gespalten wird, und das destillative Entfernen des Acetons in einer oder mehreren Apparaturen stattfindet.

18. Verfahren nach zumindest einem der Ansprüche 1 bis 17,
**dadurch gekennzeichnet,**
**dass** die Reaktion, bei welcher Wasser an das Mesityloxid angelagert und dieses in Aceton gespalten wird, und das destillative Entfernen des Acetons in einer Destillationsblase stattfindet.

19. Verfahren nach zumindest einem der Ansprüche 1 bis 17,
**dadurch gekennzeichnet,**
**dass** die Reaktion, bei welcher Wasser an das Mesityloxid angelagert und dieses in Aceton gespalten wird, in einem Reaktor oder einem lonentauscher stattfindet, und dass das destillative Entfernen des Acetons in einer Destillationskolonne stattfindet.

20. Verfahren nach zumindest einem der Ansprüche 1 bis 19,
**dadurch gekennzeichnet,**
**dass** in die Apparatur, in welcher die Reaktion, bei welcher Wasser an das Mesityloxid angelagert wird und dieses in Aceton gespalten wird, stattfindet, zumindest jeweils so viel Wasser und Mesityloxid, in Form von Mesityloxid aufweisender Phase nachdosiert werden, wie Wasser und Mesityloxid durch die Reaktion aus dem Reaktionsgemisch entfernt werden.
